(19) Europäisches Patentamt
European Patent Office
Office européen des brevets

(11) Veröffentlichungsnummer: **0 063 267**
**B1**

(12) # EUROPÄISCHE PATENTSCHRIFT

(45) Veröffentlichungstag der Patentschrift: **05.12.84**

(51) Int. Cl.³: **C 07 D 237/22**

(21) Anmeldenummer: **82102697.8**

(22) Anmeldetag: **31.03.82**

(54) **Verfahren zur Herstellung von Ameziniummetilsulfat.**

(30) Priorität: **10.04.81 DE 3114496**

(43) Veröffentlichungstag der Anmeldung: **27.10.82 Patentblatt 82/43**

(45) Bekanntmachung des Hinweises auf die Patenterteilung: **05.12.84 Patentblatt 84/49**

(84) Benannte Vertragsstaaten: **AT BE CH DE FR GB IT LI LU NL**

(56) Entgegenhaltungen: **DE - A - 1 912 941**

(73) Patentinhaber: **BASF Aktiengesellschaft, Carl-Bosch-Strasse 38, D-6700 Ludwigshafen (DE)**

(72) Erfinder: **Wiersdorff, Walter-Wielant, Dr., Blockfeldstrasse 15, D-6704 Mutterstadt (DE)**
Erfinder: **Kropp, Rudolf, Sprottauer Strasse 2, D-6703 Limburgerhof (DE)**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).



ACTORUM AG

**Beschreibung**

Die vorliegende Erfindung betrifft ein Verfahren zur Herstellung von Ameziniummetilsulfat (1-Phenyl-4-amino-6-methoxypyridazinium-methosulfat).

Das Antidepressivum Ameziniummetilsulfat ist aus der DE-AS 19 12 941 (vgl. Beispiel 1) bekannt. Es wird durch Umsetzen von 1-Phenyl-4-aminopyridazon-(6) mit Dimethylsulfat und Absaugen des Reaktionsgemisches hergestellt. Das so erhaltene Ameziniummetilsulfat ist ein dunkelbraun gefärbtes Produkt, welches Reste an Dimethylsulfat enthält und nur unter grossen Verlusten gereinigt werden kann.

Es wurde nun ein Weg gefunden, auf dem man Ameziniummetilsulfat in guter Ausbeute und Qualität erhalten kann.

Gegenstand der Erfindung ist ein Verfahren zur Herstellung von Ameziniummetilsulfat durch Umsetzen von 1-Phenyl-4-aminopyridazon-6 mit Dimethylsulfat und anschliessendes Isolieren des Ameziniummetilsulfats durch Kristallisation, dadurch gekennzeichnet, dass man nicht umgesetztes Dimethylsulfat vor der Kristallisation des Ameziniummetilsulfats an ein Amid der Formel

$$R^1\text{-}(NH)_n\text{-}CO\text{-}NR^2R^3,$$

worin
$R^1$ Wasserstoff, $C_{1-8}$-Alkyl, $C_{1-6}$-Alkoxy oder $C_{1-8}$-Alkylamino
$R^2$ und $R^3$ Wasserstoff, $C_{1-8}$-Alkyl oder
$R^1$ und $R^2$ zusammen einen $C_{3-5}$-Alkylenrest und
n die Zahl 0 oder 1 bedeuten,
wobei jedoch wenigstens einer der Reste $R^1$, $R^2$ und $R^3$ Wasserstoff bedeutet,
oder an das entsprechende Thioamid bindet.

Die Umsetzung von 1-Phenyl-4-aminopyridazon-6 mit Dimethylsulfat (DMS) geschieht in bekannter Weise. Zweckmässig wird dabei das DMS im Überschuss verwendet. Es kann aber auch mit anderen Lösungsmitteln kombiniert werden, vgl. DE-AS 19 12 941, Spalte 3, Zeilen 4 bis 10. Diese Lösungsmittel sollen jedoch — wenn überhaupt — nur in begrenzter Menge zugesetzt werden, damit die Konzentration an DMS, namentlich gegen Reaktionsende, nicht zu gering wird.

Die Reaktion kann bei Temperaturen von 0 bis 200°C ablaufen, besonders bei 30 bis 110°C, vorzugsweise jedoch zwischen etwa 60°C und 90°C. Die Reaktionszeit wird dabei so gewählt, dass die z.B. bei 120°C innerhalb einer Stunde erfolgende Zersetzung zu braunschwarzen Kristallmaischen weitgehend unterdrückt wird, beispielsweise 2 Stunden bei 70°C. Die Umsetzung kann kontinuierlich oder diskontinuierlich ausgeführt werden oder halbkontinuierlich z.B. in Kaskaden.

Gemäss der Erfindung wird dem so erhaltenen Reaktionsgemisch ein Amid zugesetzt, und zwar in einer Menge, dass der Dimethylsulfat-Überschuss sicher vernichtet wird.

Als spezielle Beispiele für Amide seien genannt: Formamid, N-Methylformamid, N-Methylacetamid, Propionsäureamid, Isobuttersäure-N-methylamid, 2-Ethylhexansäure-isopropylamid, Phenylessigsäurecyclohexylamid, Pyrrolidon, Piperidon, Caprolactam, C-Methylcaprolactam, Capryllactam, Oenanthlactam, Laurinlactam, Harnstoff, N-Methylharnstoff, N,N-Dimethylharnstoff, N,N,N'-Trimethylharnstoff, N-Methyl-N'-ethylharnstoff, N,N'-Dimethylharnstoff, N,N'-Diisopropylharnstoff, N-Methyl-N'-2-ethylhexylharnstoff, Ethylenharnstoff, Propylenharnstoff, N-Methylpropylenharnstoff, O-Methylurethan, N,O-Dimethylurethan und N,O-Diethylurethan.

Die Amide werden dem Reaktionsansatz in flüssigem oder festem Zustand oder in einem Lösungsmittel gelöst oder suspendiert zugeführt. Die Umsetzung der Amide mit DMS erfolgt in einem Temperaturbereich, der von der Konzentration, der Verweilzeit und der Reaktivität des Amids bestimmt wird, das entstehende Amezinium jedoch unverändert lässt. Der Temperaturbereich liegt bei 0 bis 200°C vorzugsweise 20 bis 120°C und ganz besonders bei 60 bis 100°C.

Die Reaktionsparameter sollten so gewählt werden, dass die Umsetzung des DMS mit dem Amid bei diskontinuierlicher Arbeitsweise zwischen 5 Minuten und 5 Stunden, vorzugsweise bei 0,5 bis 2 Stunden liegt.

Das Ameziniummetilsulfat wird aus dem Reaktionsgemisch als Festprodukt isoliert, gegebenenfalls nach Verdünnen mit Lösungsmitteln, wie die bereits in der DE-AS 19 12 941 zitierten oder Alkoholen, vorzugsweise Methanol.

Im Gegensatz zu den Verfahren der DE-AS 19 12 941 wird das Ameziniummetilsulfat nach dem neuen Verfahren frei von DMS erhalten. Das ist ein grosser Vorteil, weil das DMS wegen der Labilität des Ameziniummetilsulfats gegen Basen und andere Nucleophile nicht mit Hilfe der letzteren entfernt werden kann.

Nach dem neuen Verfahren erhält man das Ameziniummetilsulfat bereits direkt ohne weitere Reinigung in fast farbloser Form und in guten Ausbeuten.

Die folgenden Beispiele sollen die Erfindung erläutern.

*Beispiel 1*

66,35 g Dimethylsulfat werden auf 80°C erwärmt und innerhalb von 40 min mit 40 g 1-Phenyl-4-aminopyridazon-6 versetzt. Die entstehende Maische wird weitere 1,5 h bei 78 bis 82°C gerührt und dann bei 80°C innerhalb von 15 min mit 40,36 g Caprolactam, welches in 27 ml warmem Methanol gelöst ist, versetzt. Das Gemisch wird noch 1 h bei 90°C gerührt, mit 25 ml Methanol versetzt und auf 0°C abgekühlt. Die Maische wird 2 h bei 0 bis 5°C gerührt, abgesaugt und mit Methanol gewaschen. Die Ausbeute an Ameziniummetilsulfat beträgt nach dem Trocknen 50,70 g (75,7%) beigefarbenes Produkt, Fp = 173 bis 175°C (Zersetzung). Nach Umkristallisieren aus Methanol werden aus 30,00 g des erhaltenen Produktes 25,50 g (85,0%) farbloses Kristallisat erhalten, Fp = 176 bis 177°C (Zersetzung). Die Ausbeute über beide Stufen beträgt 64,3%.

*Beispiele 2 bis 10*

Das Beispiel 1 wurde mit den aus der Tabelle ersichtlichen Amiden wiederholt. Dabei wurden die

angegebenen Ausbeuten und Schmelzpunkte erreicht. Die Rohprodukte waren schwach gefärbt, während die Endprodukte fast farblos waren.

*Vergleichsbeispiel*

Setzt man die in Beispiel 1 angegebenen Mengen 1-Phenyl-4-aminopyridazon-(6) entsprechend Beispiel 1 der DE-AS 19 12 941 um, so erhält man 91% dunkelbraunes Rohprodukt, Fp 148 bis 157°C. Nach dem Umkristallisieren erhält man daraus 21% (bezogen auf 1-Phenyl-4-aminopyridazon-6) braunes Produkt, Fp 162 bis 164°C. Erst durch nochmaliges Umkristallisieren erhält man 16,6% reines Produkt, Fp 174,5 bis 175,5°C.

*Tabelle*

| Beispiel | Amid | Rohprodukt Ausbeute % | Rohprodukt Fp°C | Endprodukt Ausbeute % | Endprodukt Fp°C (Zersetzung) |
|---|---|---|---|---|---|
| 2 | Harnstoff | 78,9 | 173-174 | 87,0 | 176 -177 |
| 3 | Thioharnstoff | 72,9 | 171-173 | 85,6 | 174,5-176 |
| 4 | Formamid | 89,6 | 163-168 | 76,8 | 174 -176 |
| 5 | Pyrrolidon | 77,1 | 172-174 | 85,2 | 176,5-177,5 |
| 6 | N-Methylformamid | 78,8 | 171-172 | 85,8 | 174,5-176,5 |
| 7 | Propylenharnstoff | 83,8 | 162-164 | 83,1 | 175,5-176,5 |
| 8 | Acetamid | 80,7 | 172-173 | 89,7 | 175,5-176,5 |
| 9 | Cyclohexylharnstoff | 83,3 | 170-173 | 89,3 | 175,5-177 |
| 10 | O-Ethylurethan | 77,3 | 173-174 | 89,6 | 175,5-177 |

**Patentanspruch**

Verfahren zur Herstellung von Ameziniummetilsulfat durch Umsetzen von 1-Phenyl-4-aminopyridazon-6 mit Dimethylsulfat und anschliessendes Isolieren des Ameziniummetilsulfats durch Kristallisation, dadurch gekennzeichnet, dass man nicht umgesetztes Dimethylsulfat vor der Kristallisation des Ameziniummetilsulfats an ein Amid der Formel

$$R^1\text{-}(NH)_n\text{-}CO\text{-}NR^2R^3,$$

worin
$R^1$ Wasserstoff, $C_{1\text{-}8}$-Alkyl, $C_{1\text{-}6}$-Alkoxy oder $C_{1\text{-}8}$-Alkylamino
$R^2$ und $R^3$ Wasserstoff, $C_{1\text{-}8}$-Alkyl oder
$R^1$ und $R^2$ zusammen einen $C_{3\text{-}5}$-Alkylenrest und
n die Zahl 0 oder 1 bedeuten,
wobei jedoch wenigstens einer der Reste $R^1$, $R^2$ und $R^3$ Wasserstoff bedeutet,
oder an das entsprechende Thioamid bindet.

**Claim**

A process for the preparation of amezinium metilsulfate by reacting 1-phenyl-4-aminopyridaz-6-one with dimethyl sulfate and subsequently isolating the amezinium metilsulfate by crystallization, wherein unreacted dimethyl sulfate, before crystallization of the amezinium metilsulfate, is bonded to an amide of the formula

$$R^1\text{-}(NH)_n\text{-}CO\text{-}NR^2R^3,$$

where $R^1$ is hydrogen, $C_1$-$C_8$-alkyl, $C_1$-$C_6$-alkoxy or $C_1$-$C_8$-alkylamino, $R^2$ and $R^3$ are each hydrogen or $C_1$-$C_8$-alkyl, or $R^1$ and $R^2$ together are a $C_3$-$C_5$-alkylene radical, and n is 0 or 1, at least one of the radicals $R^1$, $R^2$ and $R^3$, however, denoting hydrogen, or to the corresponding thioamide.

**Revendication**

Procédé de préparation du métil-sulfate d'amézinium par réaction de la phényle-1 amino-4 pyridazone-6 avec du sulfate de diméthyle, suivie de l'isolement du métil-sulfate d'amézinium par cristallisation, caractérisé en ce que, préalablement à la cristallisation du métil-sulfate d'amézinium, le sulfate de diméthyle non transformé est fixé sur un amide de la formule

$$R^1\text{-}(NH)_n\text{-}CO\text{-}NR^2R^3,$$

dans laquelle
$R^1$ désigne un atome d'hydrogène, un radical alcoyle en $C_1$ à $C_8$ ou alcoxy en $C_1$ à $C_6$ ou un groupe alcoyl en ($C_1$ à $C_8$)-amino,
$R^2$ et $R^3$ désignent chacun un atome d'hydrogène ou un radical alcoyle en $C_1$ à $C_8$ ou forment ensemble un groupe alcoylène en $C_3$ à $C_5$ et
n vaut 0 ou 1,
l'un au moins des substituants $R^1$, $R^2$ et $R^3$ désignant un atome d'hydrogène,
ou sur un thio-amide correspondant.